# EUROPÄISCHE PATENTSCHRIFT

(11) **EP 1 291 696 B1**
(45) Veröffentlichungstag und Bekanntmachung des Hinweises auf die Patenterteilung: **13.10.2004**
(21) Anmeldenummer: 02102212.4
(22) Anmeldetag: 23.08.2002
(51) Int. Cl.: G02B 21/08, A61B 3/13

(54) **Prismenkonstruktion für simultane 0-Grad und Schräg-Beleuchtung eines Stereo-Operationsmikroskops**
Prism configuration for simultaneous 0 and oblique angle illumination in a stereo surgical microscope
Configuration de prismes pour une illumination normale et oblique dans un stéréomicroscope chirurgical

(30) Priorität: 07.09.2001 DE 10144067
(43) Veröffentlichungstag der Anmeldung: 12.03.2003
(73) Patentinhaber: Leica Microsystems (Schweiz) AG, 9435 Heerbrugg (CH)
(72) Erfinder: Sander, Ulrich, 9445 Rebstein (CH); Déverin, Jaques Alain, Dr., 9443 Widnau (CH)
(74) Vertreter: Rosenich, Paul, Dipl.-Ing.

(56) Entgegenhaltungen:
- WO-A-99/59016
- DE-A- 4 028 605
- DE-A- 19 538 382
- US-A- 5 627 613
- PATENT ABSTRACTS OF JAPAN vol. 1998, no. 10, 31. August 1998 (1998-08-31) -& JP 10 133122 A (NIKON CORP), 22. Mai 1998 (1998-05-22)
- PATENT ABSTRACTS OF JAPAN vol. 1997, no. 08, 29. August 1997 (1997-08-29) -& JP 09 105866 A (OLYMPUS OPTICAL CO LTD), 22. April 1997 (1997-04-22)

## Beschreibung

Die Erfindung betrifft eine Prismenkonstruktion für simultane 0°- und Schräg-Beleuchtung eines Stereo-Operationsmikroskops.

(Stereo-)Operationsmikroskope verfügen häufig über eine Beleuchtungseinspiegelung, durch die ein Beleuchtungsstrahlengang über eine Umtenkeinrichtung in ein schräges und ein 0°-Teilstrahlenbündel aufgeteilt wird, welche in den Hauptstrahlengang des Mikroskops eingeblendet werden.

Eingespiegelte Strahlengänge zur Beleuchtung eines Objektfeldes werden bei verschiedensten Anwendungen, insbesondere auch in der Ophthalmologie, angewendet. Dabei wird üblicherweise das Patientenauge durch die Beleuchtung des Operationsmikroskops direkt beleuchtet. Dadurch ist - bei zu großer Helligkeit - besonders die Netzhaut, aber auch die Kornea stark gefährdet.

Es werden deshalb immer wieder Anstrengungen unternommen, diese Gefährdung auszuschließen oder zu minimieren. Dazu wurde zum Beispiel in der US-A-4715704 eine lichtundurchlässige Blende in den Beleuchtungsstrahlengang eingeschwenkt, dessen Ort konjugiert zur Objektebene ist. Eine Blende mit räumlichem und spektralem Verlauf ist in der DE-A-101 08 254 beschrieben. Diese Blenden haben alle eine Abdunkelung des Objektfeldes zur Folge, die natürlich den besten Augenschutz bewirkt. Allerdings wird dadurch auch die Arbeit des Chirurgen durch eine eingeschränkte Sicht behindert.

Wünschenswert sind also eigentlich ein gewisser Augenschutz und eine ausreichende Beleuchtung für den Chirurgen.

Eine Lösung besteht bei einem bekannten Ophthalmo-Objektiv darin, dass die 0°-Beleuchtung über eine Drehung des Objektivs ausgeschwenkt werden kann.

Eine andere bekannte Lösung schlägt das Ausschalten der 0°-Beleuchtung mittels Shutter vor. Bei beiden Varianten wird die schädliche 0°-Beleuchtung ausgeschaltet, aber das Objektfeld weiterhin durch den sogenannten 6°-Anteil beleuchtet. In der EP 1 153 569 A2 wird ein Vorschlag gemacht, bei dem durch Ausschwenken verschiedener Linsenelemente die Abbildung des schädlichen Lichts auf der Netzhaut so verändert wird, dass die Schädlichkeit erheblich reduziert wird.

Eine weitere, aber vom optischen Aufbau aufwändige Einrichtung ist dem Zeiss-Prospekt mit dem OPMI® MDO zu entnehmen. Dort kann das Patientenauge, wenn der Rotreflex nicht mehr benötigt wird, mit einer eingebauten Schrägbeleuchtung beleuchtet werden.

In der DE-A-40 28 605 und der DE-A-196 50 773 wird jeweils ein Aufbau mit zwei Umlenkmitteln beschrieben, bei welchen das erste entweder teilweise oder ganz verschiebbar angeordnet ist. Die weiteren Umlenkmittel werden nahe bei den Beobachtungsstrahlengängen, diese ganz oder teilweise umschließend, angeordnet. Dadurch wird ein gleichmäßiger Rotreflex erhalten.

Darüber hinaus ist aus der WO-A-99/59016 eine Beleuchtungseinrichtung bekannt, bei welcher ein zur Hauptachse des Mikroskops paralleler und ein schräger Lichtstrom geschaffen werden, welche voneinander unabhängig reguliert werden können. Die Nachteile bei dieser Lösung sind: Es wird durch einen 0°-Prismen-Schnorchel nur ein Teil der Beleuchtungspupille genutzt, was zu Vignettierung und Feldbeschnitt führen kann. Ebenfalls ist das 0°-Beleuchtungsfeld kleiner als das 6°-Feld. Dies führt dazu, dass der Rotreflex unter Umständen nicht überall im Leuchtfeld auftritt. Weiterhin werden durch diese Anordnungen mindestens drei Reflexe auf der Kornea abgebildet, was zuweilen unerwünscht ist.

Weitere Beleuchtungseinrichtungen für die simultane Beleuchtung eines Objekts aus zwei unterschiedlichen Richtungen sind aus den Druckschriften JP-A-10133122, JP-A-09105866 und US-A-5627613 bekannt.

Der Erfindung liegt somit die Aufgabe zugrunde, eine Beleuchtungseinrichtung zu schaffen, welche die Lichteintrittspupille optimal nutzt und - wenn die Rotreflex-Beleuchtung des Augenhintergrundes nicht mehr benötigt wird - einen wirksamen Augenschutz für den Patienten ermöglicht, ohne die Intensität der Objektfeldbeleuchtung zu reduzieren und damit die einleitend genannten Nachteile des Standes der Technik zu vermeiden. Die Reihung der beiden Teil-Aufgaben kann auch umgekehrt werden, nämlich, dass ein wirksamer Augenschutz ermöglicht wird und außerdem die Lichteintrittspupille optimal genutzt wird.

Gelöst wird diese Aufgabe durch eine Beleuchtungseinrichtung mit den Merkmalen gemäß Anspruch 1; insbesondere durch folgende Maßnahmen:
- Das Umlenkelement ist als Prismenkombination zur Umlenkung eines ersten Beleuchtungs-Teilstrahlenbündels in Femdistanz zur optischen Achse des Hauptobjektivs (Schrägbeleuchtung) sowie zur Umlenkung eines zweiten Beleuchtungs-Teilstrahlenbündels in Nahdistanz zur optischen Achse des Hauptobjektivs (Erzielung der 0°-Beleuchtung) ausgebildet. Das Umlenkelement nimmt das Licht aus der gesamten Beleuchtungspupille auf und überdeckt somit die gesamte Fläche des ersten Umlenkelementes (6°). Damit wird außerdem eine sehr geringe Bauhöhe erreicht.
- Der Augenschutz des Patienten wird dadurch gegeben, dass die Prismenkombination mit der nachfolgenden Beleuchtungsoptik von den Beobachtungsstrahlengängen weg verschoben wird. Dies bewirkt, dass der Leuchtfleck, der auf der Netzhaut entsteht und in der Nähe der Makula schädlich sein kann, auch von diesem Ort entfernt wird. Der Chirurg verschiebt die gesamte Beleuchtungseinheit, wenn er den Rotreflex nicht mehr benötigt; er hat dann aber immer noch die gleiche Objektfeldbeleuchtung, aber jetzt ohne Rotreflex, nur mit einem anderen Winkel. Diese Verschiebung kann erfindungsgemäß manuell oder motorisch betätigt werden. Der Augenschutz kann selbstverständlich auch mit einer anderen Lichteinspiegelungs-Konstruktion kombiniert werden und umgekehrt kann die erfindungsgemäße Prismenkombination auch unabhängig von der Verschiebbarkeit sinnvoll eingesetzt werden.
- Der 0°-Schnorchel wird dadurch ersetzt, dass das 0°-Licht nicht mehr direkt zwischen die Beobachtungsstrahlengänge eingespiegelt wird, sondern etwas in Richtung der Lichtquelle verschoben. Hierzu wird gemäß einer besonderen Ausgestaltung der Erfindung eine neue Form für das 0°-Umlenkelement gewählt.

Die Situation der Beleuchtung für Mikroskope wird durch die Erfindung verbessert:
- Durch die erfindungsgemäße Einspiegelung des Beleuchtungsstrahlenganges über ein - vorzugsweise zur optischen Achse des Mikroskops verschiebbares - Umlenkelement, welches zwei optische Strahlengänge erzeugt, kann - wie an sich bekannt - gleichzeitig eine 0°-und eine schräge Beleuchtung des Objektes erzeugt werden.
- Durch die Verschiebung des Umlenkelementes einzeln oder zusammen mit der Beleuchtung radial gegen außen wird die 0°-Beleuchtung zu einer schrägen Beleuchtung, wodurch der Rotreflex vermieden wird. Die erfindungsgemäße Verschiebbarkeit ist dabei unabhängig von der Art des Einspiegelungsaufbaus. So könnte z.B. ein Aufbau gemäß der erwähnten Publikation WO 99/59016 ausgebildet sein, jedoch mit dem Unterschied, dass das Umlenkelement senkrecht zu der optischen Achse des Hauptobjektivs verschiebbar ist.
- Eine Vignettierung und ein Feldbeschnitt des Leuchtfeldes werden vermieden.
- Beide Leuchtfelder sind gleich groß und homogen ausgeleuchtet.
- Der Rotreflex ist im zentrierten Zustand über das ganze Leuchtfeld sichtbar.
- Es entstehen maximal zwei sehr nahe beieinander liegende Kornea-Reflexe.
- Das erfindungsgemäße neue 0°-Prisma gemäß der besonderen Ausgestaltung fügt sich in seiner vorgeschlagenen Form sehr nahe an die beiden stereoskopischen Beobachterstrahlengänge an, so dass insbesondere ein guter Rotreflex erzeugt werden kann.

Eine besondere Weiterentwicklung bildet die Kombination der Erfindung mit der DE-A-195 38 382, in welcher ein System beschrieben wird, bei dem durch eine automatische Entfernungsmessung - wenn ein kritischer Abstand unterschritten wird - Warnsignale abgegeben werden oder die Beleuchtung reduziert oder abgeschaltet wird. Diese Einrichtung kann dazu verwendet werden, dass sie automatisch bei kritischen Verhältnissen das Beleuchtungsprisma mit oder ohne Beleuchtungsoptik und Lampe von der Beobachtungspupille weg verschiebt.

Im obigen Text wird zwar auf ein Operationsmikroskop Bezug genommen; die Erfindung ist jedoch nicht darauf eingeschränkt, sondern sie ist auch bei anderen optischen Geräten mit Zusatz-Beleuchtungen, z.B. bei Mikroskopen zur Untersuchung von Leiterplatten, einsetzbar.

Anhand von schematischen Darstellungen wird die Erfindung beispielhaft näher erläutert. Es zeigen dabei:
- Fig. 1: eine Beleuchtungs-Einspiegelung über eine Prismenkombination in den Hauptstrahlengang eines Mikroskops;
- Fig. 2: eine Beleuchtungs-Einspiegelung mittels einer sich im zentrierten Zustand befindlichen Prismenkombination in den Hauptstrahlengang eines Mikroskops und
- Fig. 2a: eine analoge Darstellung wie Fig. 2, wobei sich die Prismenkombination im zur optischen Achse radial verschobenen Zustand befindet.

Die Figuren werden zusammenhängend und übergreifend beschrieben. Gleiche Bezugszeichen bedeuten gleiche Bauteile, Bezugszeichen mit unterschiedlichen Indizes geben funktionsgleiche Bauteile an.

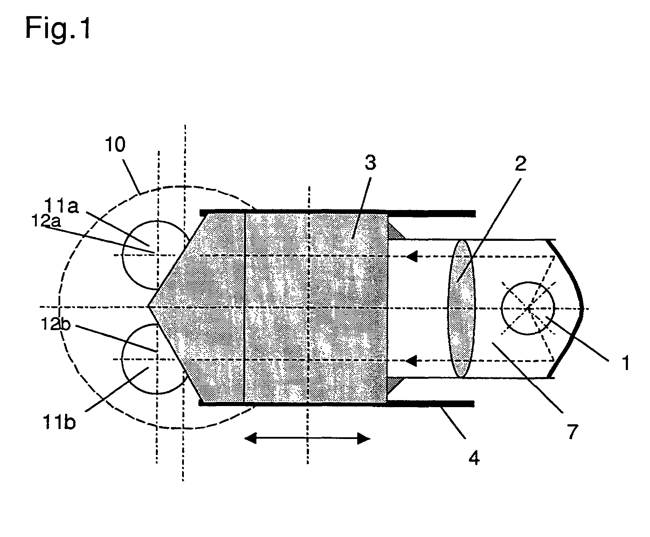

Die Fig. 1 zeigt eine Aufsicht-Darstellung einer Beleuchtungs-Einspiegelung mit einer Lichtquelle 1, einer Beleuchtungsoptik 2, einer fest mit der Lichtquelle 1 und der Beleuchtungsoptik 2 verbundenen Prismenkombination 3, einer Verschiebevorrichtung 4 für die Prismenkombination 3, einem Lichtquellen-Beleuchtungsstrahlenbündel 7, einem Hauptobjektiv 10, sowie die Beobachtungsstrahlengänge 11a, 11b.

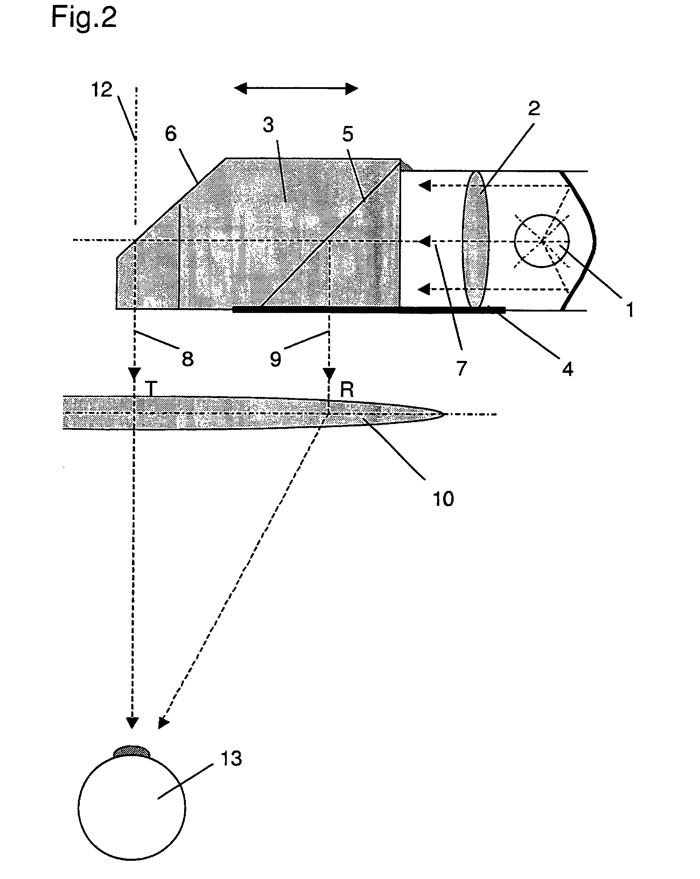

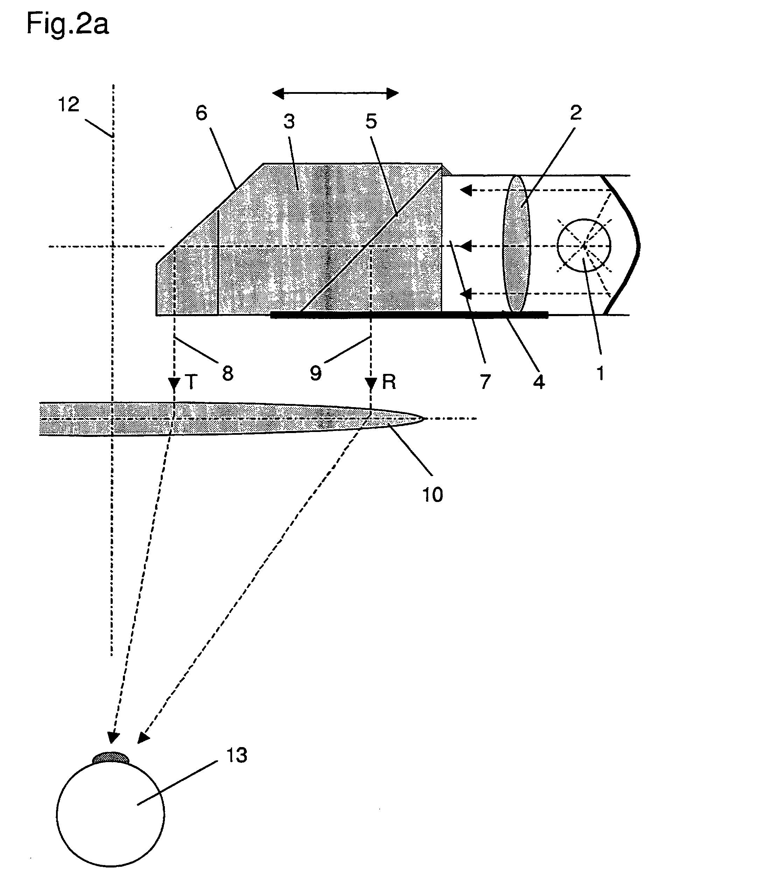

Die Fig. 2 zeigt die in Fig. 1 dargestellte Beleuchtungs-Einspiegelung als Schnittbild aus seitlicher Ansicht mit einer Lichtquelle 1, einer Beleuchtungsoptik 2, einer sich im zur optischen Achse 12 des Hauptobjektivs 10 zentriert befindlichen Prismenkombination 3 mit einer Strahlenteilerfläche 5 und einer Spiegelfläche 6, einem Beleuchtungs-Teilstrahlenbündel 8 zur 0°-Beleuchtung des Objekts 13, einem Beleuchtungs-Teilstrahlenbündel 9, welches von der optischen Achse 12 des Hauptobjektivs 10 weiter entfernt ist, sowie einem Hauptobjektiv 10.

Fig. 2a zeigt analog zu Fig. 2 die Beleuchtungs-Teilstrahlenbündel 8 und 9, wenn sich die Prismenkombination 3 in radial verschobenem Zustand gegenüber der optischen Achse 12 des Hauptobjektivs befindet.

Zur Funktionsweise wird zunächst auf Fig. 2 Bezug genommen. Beim Einsatz einer sich im zentrierten Zustand befindlichen erfindungsgemäßen Prismenkombination 3 mit einer Strahlenteilerfläche 5 und einer Spiegelfläche 6 wird das Lichtquellen-Beleuchtungsstrahlenbündel 7 in zwei zur optischen Achse 12 des Hauptobjektivs koaxiale Beleuchtungs-Teilstrahlenbündel 8 und, 9 umgelenkt. Das bedeutet, die Strahlenteilerfläche 5 bewirkt über das Hauptobjektiv 10 eine von der optischen Achse 12 des Hauptobjektivs 10 abweichende Beleuchtung des Objektes 13. Die Spiegelfläche 6 bildet den Teil des durch die Strahlenteilerfläche 5 hindurch tretenden Lichtquellen-Beleuchtungsstrahlenbündels 7 in die optische Achse 12 des Hauptobjektivs 10 ab und beleuchtet das Objekt 13 unter einem Winkel von ungefähr 0° (sogenannte "0°-Beleuchtung").

Wird nun - wie aus Fig.2a ersichtlich - die Prismenkombination 3 gemeinsam mit der mit ihr starr verbundenen Beleuchtungsoptik 2 und der Lichtquelle 1 radial zur optischen Achse 12 des Hauptobjektivs 10 gegen außen verschoben, wie es durch den Doppelpfeil angedeutet wurde, so wird für beide der erzeugten Beleuchtungs-Teilstrahlenbündel 8 und 9 eine von der optischen Achse 12 des Hauptobjektivs 10 abweichende, schräge Beleuchtung des Objekts 13 erreicht. Damit kann der Rotreflex und damit die intensive Beleuchtung des Augenhintergrundes in der Nähe der Makula vermieden werden.

Als Variante kann auch ein Aufbau gewählt werden, bei dem nur die Prismenkombination 3 - nicht aber die Beleuchtungsoptik 2 und/oder Lampe 1 - verschoben wird.

### Bezugszeichenliste

1 - Lichtquelle
2 - Beleuchtungsoptik
3 - Prismenkombination
4 - Verschiebevorrichtung
5 - Strahlenteilerfläche für schräge Beleuchtung
6 - Spiegelfläche für 0°-Beleuchtung
7 - Lichtquellen-Beleuchtungsstrahlenbündel
8 - Beleuchtungs-Teilstrahlenbündel für 0°-Beleuchtung
9 - Beleuchtungs-Teilstrahlenbündel für schräge Beleuchtung
10 - Hauptobjektiv
11 - Beobachtungsstrahlengang/-gänge
12 - Optische Achse von (10)
13 - Objekt

## Patentansprüche

1. Beleuchtungseinrichtung für eine simultane Beleuchtung eines Objekts (13) aus zwei unterschiedlichen Richtungen durch ein erstes und zweites Beleuchtungs-Teilstrahlenbündel (8, 9) für eine ein Hauptobjektiv (10) mit einer optischen Achse (12) enthaltende optische Betrachtungseinrichtung, insbesondere für ein Stereomikroskop, bestehend aus einer Lichtquelle (1) und einer Prismenkombination (3), **dadurch gekennzeichnet, dass** die Prismenkombination (3) aus einem ersten und einem zweiten Teilprisma aufgebaut sowie mit einer zwischen dem ersten rund zweiten Teilprisma liegenden Strahlenteilerfläche (5) und einer auf dem zweiten Teilprisma befindlichen Spiegelfläche (6) ausgestattet ist, dass die Strahlenteilerfläche (5) und die Spiegelfläche (6) so angeordnet sind, dass die Strahlenteilerfläche (5) zur Umlenkung eines Teils eines von der Lichtquelle (1) abgestrahlten Lichtquellen-Beleuchtungsstrahlenbündels (7) als das erste Beleuchtungs-Teilstrahlenbündel (9) in Femdistanz zur optischen Achse (12) des Hauptobjektivs (10) und die in Lichtrichtung hinter der Strahlenteilerfläche (5) liegende Spiegelfläche (6) zur gleichzeitigen Umlenkung eines Teils des restlichen Lichtquellen-Beleuchtungsstrahlenbündels (7) als das zweite Beleuchtungs-Teilstrahlenbündel (8) in Nahdistanz zur optischen Achse (12) dienen, und
dass die Prismenkombination (3) derart in radialer Richtung bezügtich der optischen Achse (12) verschiebbar ist, dass durch Wahl der Stellung der Prismenkombination (3) die Beleuchtungsrichtungen der Beleuchtungs-Teilstrahlenbündel (8, 9) nach Durchtritt durch das Hauptobjektiv (10) gemeinsam variiert werden können.

2. Beleuchtungseinrichtung nach Anspruch 1, **dadurch gekennzeichnet, dass** die optische Achse des Lichtquellen-Beleuchtungsstrahlenbündels (7) innerhalb der Prismenkombination (3) senkrecht zur optischen Achse (12) des Hauptobjektivs (10) verläuft, wobei die Strahlenteilerfläche (5) einen Winkel von 45° +/- 5' zur optischen Achse (12) des Hauptobjektivs (10) aufweist und die eine Totalreflexion des zweiten Beleuchtungs-Teilstrahlenbündels (8) bewirkende Spiegelfläche (6) einen Winkel von 45° zur optischen Achse (12) des Hauptobjektivs (10) aufweist.

3. Beleuchtungseinrichtung nach einem der vorhergehenden Ansprüche, **dadurch gekennzeichnet, dass** die Prismenkombination (3) - im Falle eines Stereomikroskops - zwischen dem Hauptobjektiv (10) und den Tubuslinsen beider stereoskopischen Beobachtungsstrahlengänge (11a, 11b) angeordnet und derart ausgebildet ist, dass sich der zu den beiden stereoskopischen Beobachtungsstrahlengängen (11a, 11b) hin gerichtete Teil der Prismenkombination (3) zur Erzielung einer optimalen und vignettierungsfreien Beobachtung zur optischen Achse (12) des Hauptobjektivs (10) hin verjüngt.

4. Beleuchtungseinrichtung nach einem der vorhergehenden Ansprüche, **dadurch gekennzeichnet, dass** die Prismenkombination (3) in Bezug auf die optische Achse (12) des Hauptobjektivs (10) radial verschiebbar angeordnet ist.

5. Beleuchtungseinrichtung nach einem der vorhergehenden Ansprüche, **dadurch gekennzeichnet, dass** die Prismenkombination (3) fest mit einer Lichtquelle (1) und einer Beleuchtungsoptik (2) verbunden ist, die zwischen einer Lichteintrittsfläche der Prismenkombination (3) und der Lichtquelle (1) angeordnet ist.

6. Beleuchtungseinrichtung nach einem der vorhergehenden Ansprüche, **dadurch gekennzeichnet, dass** die Prismenkombination (3) zusammen mit der Beleuchtungsoptik (2) und/oder der Lichtquelle (1) an der Verschiebevorrichtung (4) radial zur optischen Achse (12) verschiebbar ist.

7. Beleuchtungseinrichtung nach einem der vorhergehenden Ansprüche, **dadurch gekennzeichnet, dass** die Prismenkombination (3) in Bezug auf die optische Achse (12) des Hauptobjektivs (10) zentrierbar ist **und dass** mittels zentrierter Prismenkombination (3) durch das zweite Beleuchtungs-Teilstrahlenbündel (8) und im Beleuchtungszustand eine 0°-Beleuchtung des Objekts (13), während mit dem ersten Beleuchtungs-Teilstrahlenbündel (9) die von der optischen Achse (12) des Hauptobjektivs (10) abweichende schräge Beleuchtung erzeugbar ist.

8. Beleuchtungseinrichtung nach einem der vorhergehenden Ansprüche, **dadurch gekennzeichnet, dass** die Prismenkombination (3) in Bezug auf die optische Achse (12) dezentrierbar ist, wobei mittels dezentrierter Prismenkombination (3) durch beide Beleuchtungs-Teilstrahlenbündel (8, 9) eine von der optischen Achse (12) des Hauptobjektivs (10) abweichende schräge Beleuchtung erzeugbar ist.

9. Beleuchtungseinrichtung nach einem der vorhergehenden Ansprüche, **dadurch gekennzeichnet, dass** die Verschiebbarkeit der Prismenkombination (3) so ist, dass frei wählbare Beleuchtungswinkel des Objektes (13) einstellbar sind.

10. Beleuchtungseinrichtung nach einem der vorhergehenden Ansprüche, **dadurch gekennzeichnet, dass** die bzw. eine Prismenkombination (3) mit der bzw. mit einer Spiegelfläche (6) zur Erzielung einer 0°-Beleuchtung oder eine andere Lichtlenkvorrichtung wenigstens etwa radial zur optischen Achse (12) entlang einer Verschiebevorrichtung (4) manuell oder motorisch verschiebbar ist, so dass der auf das bzw. auf ein Objekt (13) einfallende Beleuchtungswinkel einstellbar ist.

11. Beleuchtungseinrichtung nach einem der vorhergehenden Ansprüche, **dadurch gekennzeichnet, dass** ein motorischer Antrieb zur Verschiebung der Prismenkombination (3) oder der anderen Lichtlenkvorrichtung vorgesehen und bevorzugt elektronisch ansteuerbar ist.

12. Beleuchtungseinrichtung nach Anspruch 11, **dadurch gekennzeichnet, dass** der motorische Antrieb der Prismenkombination (3) oder einer anderen Lichtlenkvorrichtung mit einer Entfernungsmessung gekoppelt ist, welche im Betriebszustand und bei zu geringem Abstand zum Objekt (13) (Patientenauge) die Prismenkombination (3) aus dem zentrierten Zustand (0°) heraus verschiebt.

## Claims

1. Illuminating device for simultaneous illumination of an object (13) from two different directions by a first and second illuminating pencil of rays (8, 9) for an optical viewing device containing a main objective (10) with an optical axis (12), in particular for a stereo microscope, consisting of a light source (1) and a prism combination (3), **characterized in that** the prism combination (3) is composed of a first and second part-prism and is equipped with a beam splitter surface (5) located between the first and second part-prisms, and a mirror surface (6) present on the second part-prism, **in that** the beam splitter surface (5) and the mirror surface (6) are arranged in such a way that the beam splitter surface (5) serves for deflecting a part of an illuminating pencil of rays (7) emitted by the light source (1), as the first illuminating pencil of rays (9) a remote distance from the optical axis (12) of the main objective (10) and the mirror surface (6) located in the direction of light behind the beam splitter surface (5) serves for the simultaneous deflection of a part of the remaining pencil of rays (7) from the light source, as the second illuminating pencil of rays (8) close to the optical axis (12), and **in that** the prism combination (3) is displaceable in the radial direction relative to the optical axis (12) in such a way that, by choosing the position of the prism combination (3), the illumination directions of the illuminating pencils of rays (8, 9) after passage through the main objective (10) can be varied together.

2. Illuminating device according to Claim 1, **characterized in that** the optical axis of the illuminating pencil of rays (7) from the light source is perpendicular to the optical axis (12) of the main objective (10) within the prism combination (3), the beam splitter surface (5) being at an angle of 45° +/- 5' to the optical axis (12) of the main objective (10) and the mirror surface (6) effecting total reflection of the second illuminating pencil of rays (8) being at an angle of 45° to the optical axis (12) of the main objective (10).

3. Illuminating device according to any of the preceding Claims, **characterized in that** the prism combination (3) - in the case of a stereo microscope-is arranged between the main objective (10) and the tube lenses of both stereoscopic observation beam paths (12a, 12b) and is formed in such a way that that part of the prism combination (3) which is directed towards the two stereoscopic observation beam paths (12a, 12b) tapers towards the optical axis (12) of the main objective (10) for achieving optimum and vignette-free observation.

4. Illuminating device according to any of the preceding Claims, **characterized in that** the prism combination (3) is arranged so as to be radially displaceable relative to the optical axis (12) of the main objective (10).

5. Illuminating device according to any of the preceding Claims, **characterized in that** the prism combination (3) is firmly connected to a light source (1) and an illuminating optical system (2) which is arranged between a light entry surface of the prism combination (3) and the light source (1).

6. Illuminating device according to any of the preceding Claims, **characterized in that** the prism combination (3), together with the illuminating optical system (2) and/or the light source (1) is displaceable on the displacement device radially relative to the optical axis (12).

7. Illuminating device according to any of the preceding Claims, **characterized in that** the prism combination (3) can be centred relative to the optical axis (12) of the main objective (10) and that 0° illumination of the object (13) can be produced by means of centred prism combination (3) by the second illuminating pencil of rays (8) and in the illumination state, while the oblique illumination deviating from the optical axis (12) of the main objective (10) can be achieved with the first illuminating pencil of rays (9).

8. Illuminating device according to any of the preceding Claims, **characterized in that** the prism combination (3) can be decentred relative to the optical axis (12), oblique illumination deviating from the optical axis (12) of the main objective (10) being producible by means of decentred prism combination (3) by both illuminating pencils of rays (8, 9).

9. Illuminating device according to any of the preceding Claims, **characterized in that** the displaceability of the prism combination (3) is such that freely selectable illumination angles of the object (13) can be set.

10. Illuminating device according to any of the preceding Claims, **characterized in that** the or a prism combination (3) with the or with a mirror surface (6) for achieving a 0° illumination or another light-deflecting device is displaceable manually or by means of a motor at least slightly radially relative to the optical axis (12) along a displacement device (4) so that the angle of incidence of the illumination on the or on an object (13) is adjustable.

11. The illuminating device according to any of the preceding Claims, **characterized in that** a motor drive is provided for displacing the prism combination (3) or the other light-deflecting device and is preferably electronically actuatable.

12. Illuminating device according to Claim 11, **characterized in that** the motor drive of the prism combination (3) or of another light-deflecting device is coupled to a distance measurement which, in the operating state and at a distance too close to the object (13) (patient's eye), displaces the prism combination (3) out of the centred state (0°).

## Revendications

1. Dispositif d'illumination pour l'illumination simultanée d'un objet (13) de deux directions différentes par un premier et un second faisceau lumineux partiel (8, 9) d'illumination pour un dispositif optique d'observation, qui comprend un objectif principal (10) ayant un axe optique (12), particulièrement pour un stéréomicroscope, comprenant une source lumineuse (1) et une combinaison de prismes (3), **caractérisé en ce que** la combinaison de prismes (3) est formée d'un premier et un second prisme partiel, et est équipée d'une surface de fractionnement des rayons (5) située entre le premier et le second prisme partiel et d'une surface de miroir (6) située au second prisme partiel, que la surface de fractionnement des rayons (5) et la surface de miroir (6) sont disposées de manière que la surface de fractionnement des rayons (5) sert à la déviation d'une partie d'un faisceau lumineux de source lumineuse (7) émis par la source lumineuse (1) comme premier faisceau lumineux partiel (9) d'illumination à grande distance de l'axe optique (12) de l'objectif principal (10), et la surface de miroir (6) située derrière la surface de fractionnement des rayons (5) sert à la déviation simultanée d'une partie du reste du faisceau lumineux de source lumineuse (7) comme second faisceau lumineux partiel (8) d'illumination à courte distance de l'axe optique (12), et que la combinaison de prismes (3) est déplaçable en direction radiale par rapport à l'axe optique (12) de manière que l'on peut varier ensemble les directions d'illumination des faisceaux lumineux partiels (8, 9) d'illumination après avoir traversé l'objectif principal (10) par sélection de la position de la combinaison de prismes (3).

2. Dispositif d'illumination selon la revendication 1, **caractérisé en ce que** l'axe optique du faisceau lumineux de source lumineuse (7) à l'intérieur de la combinaison de prismes (3) s'étend perpendiculairement à l'axe optique (12) de l'objectif principal (10), la surface de fractionnement des rayons (5) comprenant un angle de 45° ± 5' à l'axe optique (12) de l'objectif principal (10), tandis que la surface de miroir (6) provocant une réflexion totale du second faisceau lumineux partiel (8) comprend un angle de 45° à l'axe optique (12) de l'objectif principal (10).

3. Dispositif d'illumination selon une quelconque des revendications précédentes, **caractérisé en ce que** la combinaison de prismes (3), en cas d'un stéréomicroscope, est disposée entre l'objectif principale (10) et les lentilles de tubes des deux marches de rayons d'observation stéréoscopiques (12a, 12b) et est formée de manière que la partie de la combinaison de prismes (3) tournée vers les deux marches de rayons d'observation stéréoscopiques (12a, 12b) s'effile vers l'axe optique (12) de l'objectif principal (10) pour atteindre une observation optimale, exempte d'un vignettage.

4. Dispositif d'illumination selon une quelconque des revendications précédentes, **caractérisé en ce que** la combinaison de prismes (3) est disposée radialement déplaçable par rapport à l'axe optique (12) de l'objectif principal (10).

5. Dispositif d'illumination selon une quelconque des revendications précédentes, **caractérisé en ce que** la combinaison de prismes (3) est relié solidairement à une source lumineuse (1) et une optique d'illumination (2), qui est disposée entre la surface d'entrée de lumière de la combinaison de prismes (3) et la source lumineuse (1).

6. Dispositif d'illumination selon une quelconque des revendications précédentes, **caractérisé en ce que la** combinaison de prismes (3) conjointement avec l'optique d'illumination (2) et/ou source lumineuse (1) au dispositif de déplacement (4) est déplaçable radialement par rapport à l'axe optique (12).

7. Dispositif d'illumination selon une quelconque des revendications précédentes, **caractérisé en ce que** la combinaison de prismes (3) peut être centrée relativement à l'axe optique (12) de l'objectif principal (10), **et que** l'on peut produire une illumination à 0° au moyens de la combinaison centrée de prismes (3) par la second faisceau lumineux partiel (8) et en état illuminé, tandis que l'on peut produire une illumination oblique, qui diffère de l'axe optique (12) de l'objectif principal (10), par la premier faisceau lumineux partiel (9).

8. Dispositif d'illumination selon une quelconque des revendications précédentes, **caractérisé en ce que** la combinaison de prismes (3) peut être décentrée relativement à l'axe optique (12), une illumination oblique, qui diffère de l'axe optique (12) de l'objectif principal (10), étant productible au moyens de la combinaison de prismes (3) décentrée par les deux faisceaux lumineux partiels (8, 9).

9. Dispositif d'illumination selon une quelconque des revendications précédentes, **caractérisé en ce que** la possibilité d'un déplacement de la combinaison de prismes (3) est formée de manière que l'on peut ajuster des angles d'illumination de l'objet (13) d'une façon librement éligible.

10. Dispositif d'illumination selon une quelconque des revendications précédentes, **caractérisé en ce que** la ou une combinaison de prismes (3) avec la ou une surface de miroir (6) pour atteindre une illumination à 0° ou un autre dispositif de guidage de lumière est déplaçable, soit manuellement, soit par moteur, au moins environ en direction radiale par rapport à l'axe optique (12) le long d'un dispositif de déplacement (4) de manière que l'angle d'illumination faisant incidence au ou à un objet (13) peut être ajusté.

11. Dispositif d'illumination selon une quelconque des revendications précédentes, **caractérisé en ce qu'**un dispositif d'entraînement par moteur est prévu pour déplacer la combinaison de prismes (3) ou l'autre dispositif de guidage de lumière, et, de préférence, peut être commandé de façon électronique.

12. Dispositif d'illumination selon la revendication 11, **caractérisé en ce que** le dispositif d'entraînement par moteur de la combinaison de prismes (3) ou de l'autre dispositif de guidage de lumière est couplé à une télémétrie, qui en état de fonctionnement et en cas d'une distance trop courte de l'objet (13) (l'oeil du malade) déplace la combinaison de prismes (3) à l'écart de l'état centré (0°).
